(19)

Europäisches Patentamt
European Patent Office
Office européen des brevets

(11) **EP 2 223 251 B1**

(12) **EUROPEAN PATENT SPECIFICATION**

(45) Date of publication and mention
of the grant of the patent:
**18.07.2018 Bulletin 2018/29**

(21) Application number: **08806748.3**

(22) Date of filing: **10.10.2008**

(51) Int Cl.:
*G06F 19/00* *(2018.01)*       *G06F 17/00* *(2006.01)*

(86) International application number:
**PCT/GB2008/050932**

(87) International publication number:
**WO 2009/047569 (16.04.2009 Gazette 2009/16)**

(54) **METHOD AND APPARATUS FOR MONITORING A SUBSTANCE IN HUMAN OR ANIMAL BODIES**

VERFAHREN UND GERÄT ZUR ÜBERWACHUNG EINER SUBSTANZ IM MENSCHLICHEN ODER TIERISCHEN KÖRPER

METHODE ET APPAREIL POUR LA SURVEILLANCE D'UNE SUBSTANCE DANS L'ORGANISME D'UN HOMME OU D'UN ANIMAL

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **12.10.2007 GB 0719969**

(43) Date of publication of application:
**01.09.2010 Bulletin 2010/35**

(73) Proprietor: **Cambridge Enterprise Limited Trinity Lane Cambridge Cambridgeshire CB2 1TN (GB)**

(72) Inventor: **HOVORKA, Roman St. Albans AL1 3SP (GB)**

(74) Representative: **Martin, Philip John et al Marks & Clerk LLP 62-68 Hills Road Cambridge CB2 1LA (GB)**

(56) References cited:
**WO-A1-02/24065       WO-A2-2005/041103**

- **ROMAN HOVORKA ET AL: "Nonlinear model predictive control of glucose concentration in subjects with type 1 diabetes; Controlling glucose", PHYSIOLOGICAL MEASUREMENT, INSTITUTE OF PHYSICS PUBLISHING, BRISTOL, GB, vol. 25, no. 4, 1 August 2004 (2004-08-01) , pages 905-920, XP020074167, ISSN: 0967-3334, DOI: 10.1088/0967-3334/25/4/010**
- **WILINSKA M E ET AL: "Interstitial glucose kinetics in subjects with type 1 diabetes under physiologic conditions", METABOLISM, CLINICAL AND EXPERIMENTAL, W.B. SAUNDERS CO., PHILADELPHIA, PA, US, vol. 53, no. 11, 1 November 2004 (2004-11-01), pages 1484-1491, XP004609590, ISSN: 0026-0495, DOI: 10.1016/J.METABOL.2004.05.014**

**Description**

This application relates to monitoring and controlling the level of glucose in human or animal bodies.

**Background**

Insulin is secreted by the pancreas in a highly controlled fashion to maintain the plasma glucose concentration within a narrow physiological range. In type 1 diabetes, insulin is administered exogenously to mimic the basal and postprandial insulin needs. The standard therapy is based on multiple insulin injections using a combination of short and long acting insulin analogues supported by blood glucose self-monitoring. Treatment by the continuous subcutaneous insulin infusion (CSII), i.e. using insulin pumps, is on the rise.

Continuous glucose monitoring promises to improve glucose control in subjects with diabetes (D. C. Klonoff. Continuous Glucose Monitoring: Roadmap for 21st century diabetes therapy. Diabetes Care 28 (5):1231-1239, 2005). New minimally invasive and non-invasive techniques are being developed; see examples such as US Pat 5086229 and US Pat 5497772.

Supporting the development of new minimally invasive and non-invasive measurement techniques, a range of mathematical methods has been proposed to aid data processing. Data processing is confounded by measurements of the glucose sensor being normally carried out in a fluid distinct from plasma, such as in the interstitial, dermal, or tear fluid. The kinetics properties of the transfer between plasma and the measurement fluid result in a delay between plasma and the measurement fluid (K. Rebrin, G. M. Steil, W. P. Van Antwerp, and J. J. Mastrototaro. Subcutaneous glucose predicts plasma glucose independent of insulin: implications for continuous monitoring. American Journal of Physiology-Endocrinology and Metabolism 277 (3):E561-E571, 1999). Thus, the ratio between the plasma glucose and the glucose in the measurement fluid changes with time and appropriate techniques are required to calculate an estimate of plasma glucose from sensor measurements apart from filtering out the measurement error.

The extended (linearised) Kalman filter has been proposed as a suitable computational vehicle for data processing of glucose sensor signal, see US Pat 6575905, US Pat 6572545, WO02/24065 and E. J. Knobbe and B. Buckingham. "The extended Kalman filter for continuous glucose monitoring" (Diabetes Technol.Ther. 7 (1):15-27, 2005). Process noise is an integral component of the Kalman filter. The process noise represents the random disturbance which is not captured by the other model components and is distinct from the measurement error, which describes the random component of the measurement process. The characteristics of the process noise are usually obtained from retrospective analysis of experimental data. However, the characteristics and specifically the variance of the process noise may be subject to temporal variations due to physiological or life-style factors excluded from modelling. For example, following meal ingestion, the process noise will have considerably higher variance compared to that applicable to fasting conditions. The standard Kalman filter is unable to accommodate and correct for such temporal variations because it uses fixed values for the process noise.

There are other known methods for monitoring glucose levels, for example WO97/28787 uses an adaptive mathematic model and US 5,497,772 uses an enzymatic sensor to sense glucose levels.

The advancements in the field of continuous glucose sensors have stimulated the development of closed-loop systems based on combination of a continuous monitor, a control algorithm, and an insulin pump (R. Hovorka. Continuous glucose monitoring and closed-loop systems. Diabet.Med. 23 (1):1-12, 2006). This is denoted as an artificial pancreas. The original concept was introduced in late 70's, see US Pat. 4055175 and US Pat. 4464170, for example.

A wide spectrum of control algorithms has been proposed to titrate insulin in a closed-loop fashion, see a review by Parker *et al* (R. S. Parker, F. J. Doyle, III, and N. A. Peppas. The intravenous route to blood glucose control. IEEE Eng.Med.Biol.Mag. 20 (1):65-73, 2001).Two main categories have been employed, classical feedback control embodied in the proportional-integral-derivative (PID) controller (G. M. Steil, K. Rebrin, C. Darwin, F. Hariri, and M. F. Saad. Feasibility of automating insulin delivery for the treatment of type 1 diabetes. Diabetes 55 (12):3344-3350, 2006), and model predictive control (MPC) (R. Hovorka, V. Canonico, L. J. Chassin, U. Haueter, M. Massi-Benedetti, Federici M. Orsini, T. R. Pieber, H. C. Schaller, L. Schaupp, T. Vering, and M. E. Wilinska. Nonlinear model predictive control of glucose concentration in subjects with type 1 diabetes. Physiol Meas. 25 (4):905-920, 2004).

The Kalman filter was also used as part of control algorithm for closed-loop glucose control (R. S. Parker, F. J. Doyle, III, and N. A. Peppas. A model-based algorithm for blood glucose control in type I diabetic patients. IEEE Trans.Biomed.Eng 46 (2):148-157, 1999). A linearised Kalman filter was also proposed, see US Pat. 6572545. The Kalman filter or the extended Kalman filter (described above) provide computationally efficient means to track and predict glucose excursions and are used in combination with a physiologically-based glucoregulatory model represented by stochastic differential equations.

There are other known methods for controlling glucose levels, for example, US2003/ 0208113 describes a system for assisting a person to maintain blood glucose levels between predetermined limits and US 4,055,175 describes glucose control using a model based on quadratic and biquadratic equations.

Wilinska et al describe in "Interstitial glucose kinetics in subjects with Type 1 diabetes under physiologic conditions" (Metabolism, vol. 53, no. 11, 1484-1492, 2004) the dynamic relationship between interstitial glucose (IG) and plasma glucose (PG). The document describes how nine compartment models were postulated to account for temporal variations in the IG/PG ratio.

WO2005/041103 generally relates to a medical advisory system for providing recommendations to a user for actions to be taken. The system comprises input means for receiving a number of input parameters, processing means configured to determine from a least the input parameters a recommended action to be taken, and output means for presenting information about the determined recommended action to a user.

## Statements of invention

According to the invention there are provided an apparatus and a method according to claims 1 and 10. Preferable features of the invention are defined in the dependent claims. By using multiple models, the method may be considered to use an interacting multiple model strategy, i.e. a strategy in which two or more models may be defined with each model being a variation of the system model. Thus, in other words, according to another aspect of the invention, there is provided a method of real time substance monitoring in a living human or animal, the method comprising: inputting a time series of substance level measurements from a sensor, said substance level measurements being indicative of a inferred level of said substance, defining a system model which estimates said inferred substance level from said measured substance level, and applying an interacting multiple model strategy to the system model to provide a combined estimate of the inferred substance level.

The combined estimate may be a combined estimate of the current inferred substance level or a combined estimate of a future inferred substance level.

Each variation of the model may have a different process noise. As explained previously, the process noise represents the random disturbance which is not captured by the other model components. For each model, an estimate of the inferred substance level and the process noise is calculated in a computationally efficient manner. The estimates for each model are combined to provide a combined estimate of the inferred substance level. When combining the estimates, each estimate is preferably weighted according to an associated mixing probability, i.e. a probability of the estimate representing the true value. In other words, if one model is the most likely, this model is given the highest weighting so that the combined estimate is based primarily on this model. If no one model is the most likely, the weightings may be adjusted accordingly to give a more balanced representation of each model in the overall estimate. Said mixing probability may be updated responsive to a difference between said predicted glucose level measurement of each respective said model and said glucose level measurement from said sensor.

As an alternative to defining the models to have different process noise, the multiple models defined by the interacting multiple model strategy may differ in certain model constants. As described in the prior art, the extended Kalman filter may be used for a model which is non-linear in a certain parameter. However, the extended Kalman filter approximates the solution and for highly non-linear models this may be a problem. Using the interacting multiple model strategy permits the definition of a number of models, each differing in the fixed level of the parameter causing non-linearity. These models run in parallel, with no approximations being necessary (albeit the models are confined to discrete levels of the non-linear parameter) and the most appropriate parameter level may be chosen.

The interacting multiple model (IMM) strategy has been introduced to allow computationally efficient tracking of a maneuvering target (E. Mazor, A. Averbuch, Y. Bar-Shalom, and J. Dayan. Interacting multiple model methods in target tracking: A survey. IEEE Transactions on Aerospace and Electronic Systems 34 (1):103-123, 1998). A maneuvering target is characterised by temporal variability in acceleration, where the acceleration is, in effect, the process noise.

Besides use in radar and GPS tracking, see for example US Pat 5325098, US Pat 7079991, and US Pat. 6876925, in the biomedical field the IMM approach has been used to monitor kinetic parameters (D. S. Bayard and R. W. Jelliffe. A Bayesian approach to tracking patients having changing pharmacokinetic parameters. J.Pharmacokinet.Pharmacodyn. 31 (1):75-107, 2004) and the imaging field, see for example (P. Abolmaesumi and M. R. Sirouspour. An interacting multiple model probabilistic data association filter for cavity boundary extraction from ultrasound images. IEEE Trans.Med.Imaging 23 (6):772-784, 2004).

The present applicant has recognised that the IMM is well suited to handle the temporal variations of the process noise of substance monitoring, for example when using a Kalman filter described above.

For glucose monitoring, the inferred glucose level may be the plasma glucose concentration which is not directly measurable and the measured glucose level may be the interstitial glucose level. The glucose level may be measured intravenously, subcutaneously and/or intradermally.

The glucoregulatory model may comprise five sub-models, the sub-model of insulin absorption, the sub-model of insulin action, the sub-model of gut absorption, the sub-model of glucose kinetics, and the sub-model of interstitial glucose kinetics. Alternatively, the glucoregulatory model may consist of a sub-set of the five models, e.g. the sub-model of glucose kinetics and the sub-model of interstitial glucose kinetics which interact to predict the inferred glucose level, e.g. plasma glucose concentration, from the measured glucose level, e.g. glucose level in the interstitial fluid.

For glucose monitoring, the process noise may represent the change in the unexplained glucose influx. The interacting multiple model strategy may comprise defining for each variation of the model, a state vector comprising a set of variables each having an associated uncertainty. The set of variables may comprise variables representing glucose amounts in the accessible, non-accessible and/or interstitial compartments and a variable representing an unexplained change in glucose level. In an example, the substance being monitored may be the depth of anaesthesia. More information on the control of anaesthesia and the appropriate models which may be used in the interacting multiple model strategy may be determined from EP 1278564 and related application EP 1725278 to Aspect Medical Systems Inc. Other references are D. A. Linkens and M. Mahfouf. "Generalized Predictive Control with Feedforward (Gpcf) for Multivariable Anesthesia." International Journal Of Control 56 (5):1039-1057, 1992, M. Mahfouf and D. A. Linkens. "Non-linear generalized predictive control (NLGPC) applied to muscle relaxant anaesthesia." International Journal Of Control 71 (2):239-257, 1998, M. M. Struys, E. P. Mortier, and Smet T. De. "Closed loops in anaesthesia." Best.Pract.Res.Clin.Anaesthesiol. 20 (1):211-220, 2006 or V Sartori, P Schumacher, T Bouillon, M Luginbuehl and M Morari "On-line estimation of propofol pharmacodynamic parameters." Annual International Conference of the IEEE Engineering in Medicine and Biology Society (2005), 1 74-7.

The method may comprise applying a Kalman filter. A Kalman filter has two distinct steps; a predict step which uses the state estimate from the previous timestep to produce an estimate of the state at the current timestep and an update step which comprises using measurements at the current timestep to refine the estimate from the predict step to arrive at a new, more accurate, state estimate for the current timestep. In other words, the method may comprise predicting the state estimate from the previous timestep to produce an estimate of the state at the current timestep and updating the estimate using measurements at the current timestep to refine the estimate from the predicting step to arrive at an updated state estimate for the current timestep. The covariance, i.e. a measure of the estimated accuracy of the state estimate, may be used when refining the estimate. The update step may also comprise updating the covariance.

The method may further comprise predicting an estimate for the mixing probability and updating the predicted estimate for the mixing probability based on measurements of the substance levels. The combined estimate preferably uses the updated mixing probability estimates.

The method may further comprise an interact step which links the various models. The interact step may comprise determining the mixing probability from the mode probability, i.e. the probability that the system model transitions from one variation model to another variation model.

The invention further provides processor control code to implement the above mentioned method, in particular on a data carrier such as a disk, CD- or DVD-ROM, programmed memory such as read-only memory (Firmware), or on a data carrier such as an optical or electrical signal carrier. Code (and/or data) to implement embodiments of the invention may comprise source, object or executable code in a conventional programming language (interpreted or compiled) such as C, or assembly code, code for setting up or controlling an ASIC (Application Specific Integrated Circuit) or FPGA (Field Programmable Gate Array), or code for a hardware description language such as Verilog (Trade Mark) or VHDL (Very high speed integrated circuit Hardware Description Language). As the skilled person will appreciate such code and/or data may be distributed between a plurality of coupled components in communication with one another.

**Figures**

Figure 1a is a schematic drawing showing how the interacting multiple model strategy is used to estimate glucose level;
Figure 1b is a schematic drawing which shows how the interacting multiple model strategy is used to estimate glucose level and to determine a dose based on this estimated level;
Figure 2 is a flowchart showing the steps involved in the use of interacting multiple model strategy for state and covariance estimation;
Figure 3 is a schematic drawing of an artificial pancreas for controlling glucose levels in a patient using the interacting multiple model strategy

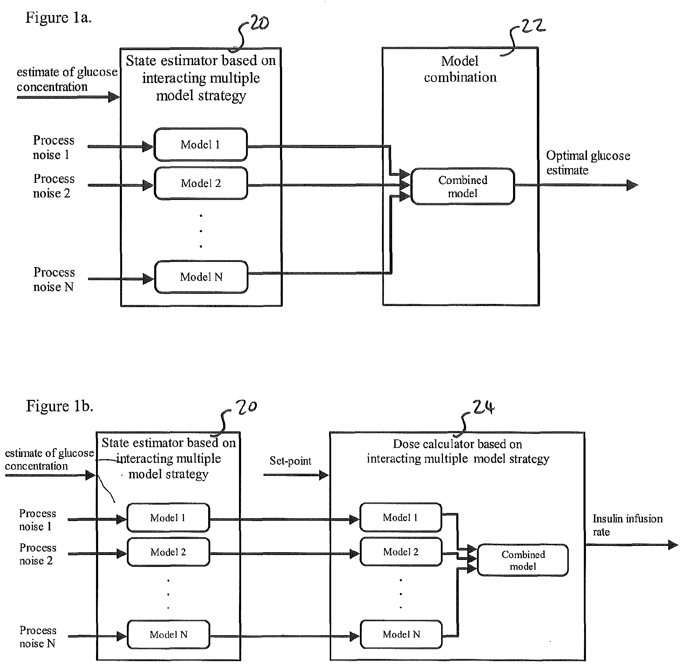

Figure 1a shows the key steps in using a multiple model strategy which is used to improve model tracking. The model used is a glucoregulatory model. Overall, N models are defined in a state estimator 20, with each model being a variation of the glucoregulatory model having a different process noise. As explained previously, the process noise represents the random disturbance which is not captured by the other model components and is distinct from the measurement error, which describes the random component of the measurement process. The characteristics of the process noise are usually obtained from retrospective analysis of experimental data. However, the characteristics and specifically the variance of the process noise may be subject to temporal variations. For each model an estimate of the state vector based on glucose measurements and the process noise is calculated in a computationally efficient manner. As an alternative to defining the models to have different process noise, the multiple models defined by the interacting multiple model strategy may differ in certain model constants.

The glucoregulatory may comprise five sub-models, the sub-model of insulin absorption, the sub-model of insulin action, the sub-model of gut absorption, the sub-model of glucose kinetics, and the sub-model of interstitial glucose kinetics. Alternatively, the glucoregulatory model may consist of a sub-set of the models described, e.g. the sub-model of glucose kinetics and the sub-model of interstitial glucose kinetics.

The *insulin absorption sub-model* is described by a two compartment (two depot) model

$$\frac{di_1(t)}{dt} = -\frac{1}{t_{xI}} i_1(t) + \frac{u(t)}{60} + \delta_{t_j}(t) v(t) \qquad (1)$$

$$\frac{di_2(t)}{dt} = -\frac{1}{t_{xI}} \left[ i_2(t) - i_1(t) \right] \qquad (2)$$

$$i(t) = \frac{1}{t_{xI}} \frac{1000}{MCR_I W} i_2(t) \qquad (3)$$

where $i_1(t)$ and $i_2(t)$ is the amount of insulin in the two subcutaneous insulin depots (U), $i(t)$ is the plasma insulin concentration (mU/1), $u(t)$ denotes insulin infusion (U/h), $v(t)$ denotes insulin boluses given at time $t_j$ (U), $t_{max,I}$ is the time-to-peak of insulin absorption (min), $MCR_I$ is the metabolic clearance rate of insulin (L/kg/min), and $W$ is subject's weight (kg).

The *insulin action sub-model* is described as

$$\frac{dr_D(t)}{dt} = p_{2D} \left( i(t) - r_D(t) \right) \qquad (4)$$

$$\frac{dr_E(t)}{dt} = p_{2E} \left( i(t) - r_E(t) \right) \qquad (5)$$

where $\gamma_D$ and $\gamma_{EGP}$ are the remote insulin actions affecting glucose disposal and endogenous glucose production, respectively, (mU/l), and $p_{2,D}$ and $p_{2,EGP}$ are the fractional disappearance rates associated with the remote insulin actions (/min).

The *gut absorption sub-model* is described by a two compartment model

$$\frac{da_1(t)}{dt} = -\frac{1}{t_{xG}} a_1(t) + v_G(t) \qquad (6)$$

$$\frac{da_2(t)}{dt} = -\frac{1}{t_{xG}} \left[ a_2(t) - a_1(t) \right] \qquad (7)$$

$$u_A(t) = \frac{5.551}{W t_{xG}} a_2(t) \qquad (8)$$

where $a_1(t)$ and $a_2(t)$ is the amount of glucose in the two absorption compartments (g), $u_A(t)$ is the gut absorption rate (mmol/kg/min), $v_G(t)$ denotes meal ingestion (g/min), and $t_{max,G}$ is the time-to-peak of the gut absorption (min).

The *glucose kinetics sub-model* is described as

$$\frac{dq_1(t)}{dt} = -\left(S_{ID}x_D(t) + k_{21}\right)q_1(t) + k_{12}q_2 - F_{01} \quad (9)$$
$$+ EGP(t) + Fu_A(t) + u_S(t)$$

$$\frac{dq_2(t)}{dt} = +k_{21}q_1(t) - k_{12}q_2(t) \quad (10)$$

$$g_P(t) = \frac{q_1(t)}{V_G} \quad (11)$$

where $q_1(t)$ and $q_2(t)$ are the masses of glucose in the accessible and non-accessible glucose compartments (mmol/kg), $k_{21}$ and $k_{12}$ are the fractional transfer rates (/min), $F_{01}$ is the non-insulin dependent glucose utilisation (mmol/kg/min), $EGP(t)$ is the endogenous glucose production (mmol/kg/min), $S_{I,D}$ is peripheral insulin sensitivity (/min per mU/l), $F$ is glucose bioavailability (unitless), $u_S(t)$ is unexplained glucose influx (mmol/kg/min), $g_P(t)$ is plasma glucose concentration, $V_G$ is the glucose distribution volume in the accessible compartment (1/kg).

The EGP is obtained as

$$EGP(t) = EGP_B \exp\left(-(r_E(t) - BIC)\frac{\ln(2)}{I_{1/2}}\right) \quad (12)$$

where $EGP_B$ is the basal endogenous glucose production (mmol/kg/min), $BIC$ is (basal) plasma insulin concentration resulting in plasma glucose concentration of 5.5mmol/l (mU/l), and $I_{1/2}$ is an increment in the plasma insulin concentration halving the EGP (mU/l).

The change in unexplained glucose influx $u_S(t)$ (the process noise) is described by a stochastic differential equation

$$du_S(t) = dw(t) \quad (13)$$

where $w(t)$ is 1-dimensional driving Wiener process.

The basal insulin concentration BIC is calculated from the basal insulin requirement (BIR; U/h) as

$$BIC = \frac{1000}{60}\frac{BIR}{MCR_I W} \quad (14)$$

where $MCR_I$ is the metabolic clearance rate of insulin (l/kg/min) and $W$ is subject's weight (kg).

The *interstitial glucose kinetics sub-model* is described as

$$\frac{dq_3(t)}{dt} = -k_{31}\left(q_3(t) - q_1(t)\right) \quad (15)$$

$$g_{IG}(t) = \frac{q_3(t)}{V_G} \quad (16)$$

where $q_3(t)$ is the mass of glucose in the interstitial fluid (mmol/kg), $k_{31}$ is the fractional transfer rate (/min), and $g_{IG}(t)$ is interstitial glucose concentration.

As shown in Figure 1a, a time series of glucose level measurements are inputted from a glucose sensor to the state estimator 20. These measurements may themselves be regarded as estimates for the plasma glucose concentration since they measure interstitial glucose levels. The state estimator 20 applies each model to calculate an estimate for the plasma glucose concentration. These estimates are combined in a modal combiner 22 to generate an optimal glucose estimate.

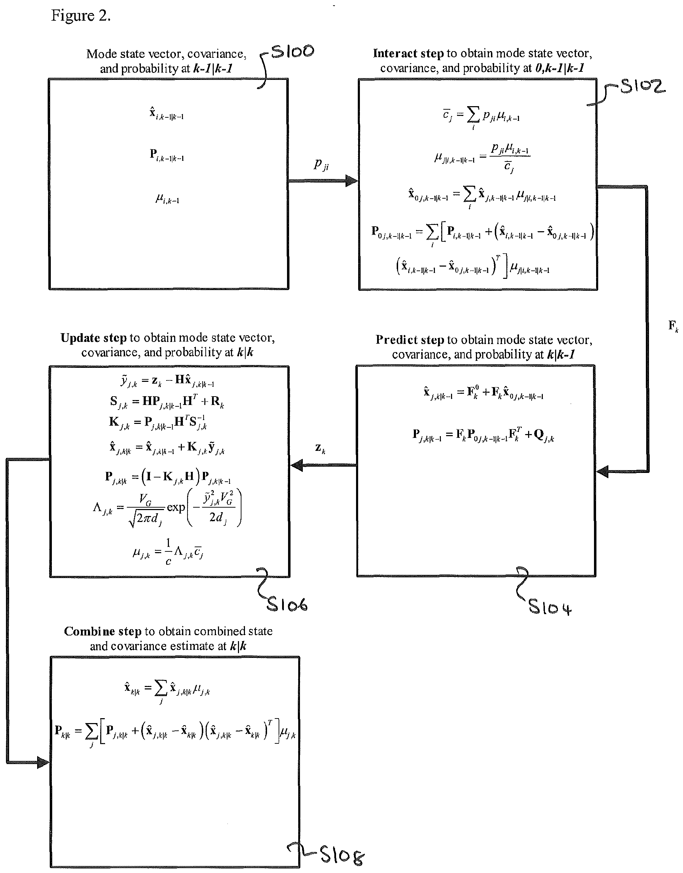

Figure 1b shows a variation of the system of Figure 1a in which the model combiner is replaced by a dose calculator 24. The estimates from the state estimator are inputted to and combined in the dose calculator 24 to generate the optimal glucose estimate. A set-point representing the desired level of plasma glucose is also inputted to the dose calculator. The dose calculator 24 inputs the optimal glucose estimate into a control law to determine the dose to the applied so that a patient has the set-point level of plasma glucose. The calculated dose is the output insulin infusion rate More details of the strategy are shown in Figure 2. The first step S100 is to define at time k-1, for each model i, a mode state vector $\mathbf{x}_{i,k-1\|k-1}$, a covariance $\mathbf{P}_{i,k-1\|k-1}$ and a mixing probability $\mu_{i,k-1\|k-1}$. The covariance is a measure of the estimated accuracy of the state estimate. The mixing probability is the probability of each mode state vector being the true state vector and is the weighting attached to each state vector estimate when calculating the final state estimate.

The extended state vector $\mathbf{x_k}$ which uses all the sub-models defined above includes nine states

$$\mathbf{x}_k^e = \left( i_{1,k}, i_{2,k}, r_{D,k}, r_{E,k}, a_{1,k}, a_{2,k}, q_{1,k}, q_{2,k}, q_{3,k}, u_{S,k} \right)^T \quad (17)$$

where $i_{1,k}$ and $i_{2,k}$ is the amount of insulin in the two subcutaneous insulin depots at time k, $\gamma_{D,k}$ and $\gamma_{E,k}$ are the remote insulin actions affecting glucose disposal and endogenous glucose production, $a_{1,k}$ and $a_{2,k}$ are the amount of glucose in the two absorption compartments, $q_{1,k}$, $q_{2,k}$, $q_{3,k}$ represent glucose amounts in the accessible, non-accessible, and interstitial compartments and $u_{s,k}$ is the unexplained glucose influx (process noise).

The function $f$ is used to calculate the predicted state $\mathbf{x_k}$ from the previous estimate $\mathbf{x_{k-1}}$

$$\mathbf{x_k} = f\left( \mathbf{x_{k-1}}, u_k, \mathbf{w_k} \right) \quad (18)$$

where $\mathbf{w_k}$ is a 1-dimensional driving Wiener process (see 13)

$$z_k = h(\mathbf{x_k}, \mathbf{v_k}) \quad (19)$$

$z_k$ is the measurement at time k of the true state $x_k$

h is the observation model which maps the true state space into the observed space and

$v_k$ is the observation noise which is assumed to be zero mean Gaussian white noise with covariance $R_k$ (see also eqn 60 below)

The state transition for $i_{1,k}$ (i.e. the transition from time k-1 to time k) is defined by the following expressions

$$i_{1,k} = f_{10} + f_{11}i_{1,k-1} \quad (20)$$

where

$$f_{10} = \frac{u_k t_{\mathrm{x}I,k-1}}{60} \left( 1 - e^{-\frac{\Delta t_k}{t_{\mathrm{x}I,k-1}}} \right) \quad (21)$$

$$f_{11} = e^{-\frac{\Delta t_k}{t_{\mathrm{x}I,k-1}}} \quad (22)$$

The state transition for $i_{2,k}$ is defined by the following expressions

$$i_{2,k} = f_{20} + f_{21}i_{1,k-1} + f_{22}i_{2,k-1} \quad (23)$$

where

$$f_{20} = f_{10} - f_{11} \frac{u_k \Delta t_k}{60} \qquad (24)$$

$$f_{21} = f_{11} \frac{\Delta t}{t_{xI,k-1}} \qquad (25)$$

$$f_{22} = f_{11} \qquad (26)$$

The state transition for $r_{D,k}$ is defined by the following expressions

$$r_{D,k} = f_{30} + f_{31}i_{1,k-1} + f_{32}i_{2,k-1} + f_{33}r_{D,k-1} \qquad (27)$$

$$f_{30} = \begin{cases} \dfrac{50u_k}{3MCR_I W}\left[1 - \dfrac{f_{33} + p_{2D}f_{11}\left(p_{2D}t_{xI,k-1}(\Delta t_k + t_{xI,k-1}) - \Delta t_k - 2t_{xI,k-1}\right)}{\left(p_{2D}t_{xI,k-1} - 1\right)^2}\right] & \text{if } p_{2D} \neq \dfrac{1}{t_{xI,k-1}} \\[20pt] \dfrac{50u_k}{3MCR_I W}\left[1 - f_{11}\dfrac{t_{xI,k-1}^2 + \left(\Delta t + t_{xI,k-1}\right)^2}{2t_{xI,k-1}^2}\right] & \text{otherwise} \end{cases}$$

$$(28)$$

$$f_{31} = \begin{cases} \dfrac{1000p_{2,D}}{MCR_I W\left(p_{2D}t_{xI,k-1} - 1\right)^2}\left(f_{33} + f_{11}\dfrac{p_{2D}t_{xI,k-1}\Delta t_k - \Delta t_k - t_{xI,k-1}}{t_{xI,k-1}}\right) & \text{if } p_{2D} \neq \dfrac{1}{t_{xI,k-1}} \\[20pt] \dfrac{500\Delta t_k^2 f_{11}}{MCR_I W t_{xI,k-1}^3} & \text{otherwise} \end{cases}$$

$$(29)$$

$$f_{32} = \begin{cases} \dfrac{1000p_{2D}}{MCR_I W\left(p_{2D}t_{xI,k-1} - 1\right)}\left(f_{11} - f_{33}\right) & \text{if } p_{2D} \neq \dfrac{1}{t_{xI,k-1}} \\[20pt] \dfrac{1000\Delta t_k f_{11}}{MCR_I W t_{xI,k-1}^2} & \text{otherwise} \end{cases} \qquad (30)$$

$$f_{33} = e^{-p_{2D}\Delta t_k} \qquad (31)$$

The state transition for $r_{E,k}$ is defined by the following expressions

$$r_{E,k} = f_{40} + f_{41}i_{1,k-1} + f_{42}i_{2,k-1} + f_{44}r_{E,k-1} \qquad (32)$$

$$f_{40} = \begin{cases} \dfrac{50u_k}{3MCR_I W}\left[1 - \dfrac{f_{44} + p_{2E,k-1}f_{11}\left(p_{2E,k-1}t_{xI,k-1}(\Delta t_k + t_{xI,k-1}) - \Delta t_k - 2t_{xI,k-1}\right)}{\left(p_{2E,k-1}t_{xI,k-1} - 1\right)^2}\right] & \text{if } p_{2E,k-1} \neq \dfrac{1}{t_{xI,k-1}} \\[4mm] \dfrac{50u_k}{3MCR_I W}\left[1 - f_{11}\dfrac{t_{xI,k-1}^2 + \left(\Delta t + t_{xI,k-1}\right)^2}{2t_{xI,k-1}^2}\right] & \text{otherwise} \end{cases}$$

$$(33)$$

$$f_{41} = \begin{cases} \dfrac{1000p_{2E,k-1}}{MCR_I W\left(p_{2E,k-1}t_{xI,k-1} - 1\right)^2}\left(f_{44} + f_{11}\dfrac{p_{2E,k-1}t_{xI,k-1}\Delta t_k - \Delta t_k - t_{xI,k-1}}{t_{\max,I}}\right) & \text{if } p_{2E,k-1} \neq \dfrac{1}{t_{xI,k-1}} \\[4mm] \dfrac{500\Delta t_k^2 f_{11}}{MCR_I W t_{xI,k-1}^3} & \text{otherwise} \end{cases}$$

$$(34)$$

$$f_{42} = \begin{cases} \dfrac{1000p_{2E,k-1}}{MCR_I W\left(p_{2E,k-1}t_{xI,k-1} - 1\right)}\left(e^{-\frac{\Delta t_k}{t_{xI,k-1}}} - e^{-p_{2E,k-1}\Delta t_k}\right) & \text{if } p_{2E,k-1} \neq \dfrac{1}{t_{xI,k-1}} \\[4mm] \dfrac{1000\Delta t_k e^{-\frac{\Delta t_k}{t_{xI,k-1}}}}{MCR_I W t_{xI,k-1}^2} & \text{otherwise} \end{cases} \qquad (35)$$

$$f_{44} = e^{-p_{2E,k-1}\Delta t_k} \qquad (36)$$

The state transition for $a_{1,k}$ is defined by the following expressions

$$a_{1,k} = f_{55}a_{1,k-1} \qquad (37)$$

where

$$f_{55} = e^{-\frac{\Delta t_k}{t_{xG,k-1}}} \qquad (38)$$

The state transition for $a_{2,k}$ is defined by the following expressions

$$a_{2,k} = f_{65}a_{1,k-1} + f_{66}a_{2,k-1} \qquad (39)$$

where

$$f_{65} = f_{55} \frac{\Delta t}{t_{xG,k-1}} \qquad (40)$$

$$f_{66} = f_{55} \qquad (41)$$

The state transitions for $q_{1,k}$, $q_{2,k}$, and $q_{3,k}$ are defined by the following expressions

$$q_{1,k} = f_{70} + f_{77}q_{1,k-1} + f_{78}q_{2,k-1} + f_{79}u_{S,k-1} \qquad (42)$$

$$q_{2,k} = f_{80} + f_{87}q_{1,k-1} + f_{88}q_{2,k-1} + f_{89}u_{S,k-1} \qquad (43)$$

$$q_{3,k} = f_{12,0} + f_{12,7}q_{1,k-1} + f_{12,8}q_{2,k-1} + f_{12,12}q_{3,k-1} + f_{12,9}u_{S,k-1} \qquad (44)$$

where coefficients $f_{70}$, $f_{77}$, $f_{78}$, $f_{79}$, $f_{80}$, $f_{87}$, $f_{88}$, $f_{89}$, $f_{12,0}$, $f_{12,7}$, $f_{12,8}$, $f_{12,9}$, and $f_{12,12}$ can be obtained algebraically as described in Appendix or by numerical approximations.
The state transitions for $u_{S,K}$ is an identity, i.e.

$$u_{S,k} = u_{S,k-1} \qquad (45)$$

Considering a subset state vector $\mathbf{x}_k$, which includes states with associated uncertainty

$$\mathbf{x}_k = \left( q_{1f,k}, q_{2f,k}, u_{S,k}, F_k, q_{3f,k} \right)^T \qquad (46)$$

where $q_{1f,x}$, $q_{2f,k}$, and $q_{3f,k}$ represent glucose amounts in the accessible, non-accessible, and interstitial compartments excluding the contribution from meals (more correctly the last meal), $u_{s,k}$ is (as before) the unexplained glucose influx (process noise) and $F_k$ is the state transition model which is applied to the previous state $x_{k-1}$ (see eqn 55).
The total amount of glucose in the compartments is calculated as

$$q_{1,k} = q_{1f,k} + q_{1m,k} \qquad (47)$$

$$q_{2,k} = q_{2f,k} + q_{2m,k} \qquad (48)$$

$$q_{3,k} = q_{3f,k} + q_{3m,k} \qquad (49)$$

where $q_{1m,k}$, $q_{2m,k}$, and $q_{3m,k}$ represent glucose amounts in the accessible, non-accessible, and interstitial compartments due to the (last) meal.
The glucose masses due to the meal are calculated as

$$q_{1m,k} = F_k f_{7,10} \qquad (50)$$

$$q_{2m,k} = F_k f_{8,10} \qquad (51)$$

$$q_{3m,k} = F_k f_{12,10} \qquad (52)$$

The state transition is obtained as

$$\mathbf{x}_k = \mathbf{F}_k^0 + \mathbf{F}_k \mathbf{x}_{k-1} + \mathbf{G}_k w_k \qquad (53)$$

where

$\mathbf{F}_k^0$ is the additive transition model which is independent of the previous state $\mathbf{x}_{k-1}$ and the process noise

$$\mathbf{F}_k^0 = \begin{bmatrix} f_{70} \\ f_{80} \\ 0 \\ 0 \\ f_{12,0} \end{bmatrix} \qquad (54)$$

and

$\mathbf{F}_k$ is the state transition model which is applied to the previous state $\mathbf{x}_{k-1}$

$$\mathbf{F}_k = \begin{bmatrix} f_{77} & f_{78} & f_{79} & 0 & 0 \\ f_{87} & f_{88} & f_{89} & 0 & 0 \\ 0 & 0 & f_{11,11} & 0 & 0 \\ 0 & 0 & 0 & 1 & 0 \\ f_{12,7} & f_{12,8} & f_{12,9} & 0 & f_{12,12} \end{bmatrix} \qquad (55)$$

and

$\mathbf{G}_k$ is the additive transition model which is applied to the process noise $w_k$

$$\mathbf{G}_k = \begin{bmatrix} g_1 \Delta t_k \\ g_2 \Delta t_k \\ \Delta t_k \\ 0 \\ g_5 \Delta t_k \end{bmatrix} \qquad (56)$$

and the change in the unexplained glucose influx (process noise) $w_k$ is normally distributed, with zero mean and standard deviation

$$\sigma_{w,k} = \sigma_w / \sqrt{\Delta t_k} \ .$$

If a glucose measurement is not made in time instance $t_k$, the unexplained glucose influx is regressed towards zero with a half-time $m_{1/2}$

$$f_{11,11} = \exp\left( -\Delta t_k \frac{\ln(2)}{m_{1/2}} \right) \qquad (57)$$

otherwise

$$f_{11,11} = 1 \qquad (58)$$

We find that the covariance $\mathbf{Q_k}$ of the unexplained glucose influx is

$$\mathbf{Q}_k = \text{cov}\left(\mathbf{G}_k w_k\right) = \sigma_{w,k}^2 \mathbf{G}_k \mathbf{G}_k^T \qquad (59)$$

At each time interval, a measurement $\mathbf{z_k}$ of interstitial glucose concentration is made. This measurement is noisy measurement of the plasma glucose. The measurement noise is normally distributed with mean 0 and standard deviation $\sigma_{Z,k}$

$$\mathbf{z}_k = \mathbf{H}\mathbf{x}_k + \mathbf{v}_k \qquad (60)$$

where

**H** is the observation model which maps the true state space into the observed space

$$\mathbf{H} = \begin{bmatrix} 0 & 0 & 0 & f_{12,10}/V_G & 1/V_G \end{bmatrix} \qquad (61)$$

and
$\mathbf{v}_k$ is the observation noise which is assumed to be zero mean Gaussian white noise with covariance $\mathbf{R_k}$

$$\mathbf{R}_k = \text{E}\left[\mathbf{v}_k \mathbf{v}_k^T\right] = \begin{bmatrix} \sigma_{Z,k}^2 \end{bmatrix} \qquad (62)$$

The initial starting position is assumed identical to the first glucose measurement $\tilde{g}_{IG,0}$ and an *apriori* bioavailability $\tilde{F}$

$$\hat{\mathbf{x}}_{0|0} = \begin{bmatrix} \tilde{g}_{IG,0} V_G \\ \tilde{g}_{IG,0} V_G \dfrac{k_{21}}{k_{12}} \\ 0 \\ F \\ \tilde{g}_{IG,0} V_G \end{bmatrix} \qquad (63)$$

The uncertainty in glucose concentration and bioavailability is expressed in the initial value of the covariance matrix $\mathbf{P_{0|0}}$

$$\mathbf{P}_{0|0} = \begin{bmatrix} \sigma_{Z,0}^2 V_G^2 & 0 & 0 & 0 & 0 \\ 0 & \sigma_{Z,0}^2 V_G^2 \dfrac{k_{21}^2}{k_{12}^2} & 0 & 0 & 0 \\ 0 & 0 & 4EGP_B^2 & 0 & 0 \\ 0 & 0 & 0 & \sigma_F^2 & 0 \\ 0 & 0 & 0 & 0 & \sigma_{Z,0}^2 V_G^2 \end{bmatrix} \qquad (64)$$

where $\sigma_F^2$ is the variance of the *apriori* distribution of bioavailability *F*.

A multiple model strategy is used to improve model tracking. Overall, *N* models are defined, which differ in the standard deviation of the unexplained glucose influx $w_k$

$$\sigma_{w1,k} = \sigma_{w1} / \sqrt{\Delta t_k}$$
$$\sigma_{w2,k} = \sigma_{w2} / \sqrt{\Delta t_k} \qquad (65)$$
$$\ldots$$
$$\sigma_{wN,k} = \sigma_{wN} / \sqrt{\Delta t_k}$$

where $\sigma_{w(i-1)} < \sigma_{wi}$ without loss of generality.

The Markov transition probability from mode (or model) *i* to *j* is defined as $p_{ji}$, i.e. the probability that the system will switch from model i to model j is $p_{ji}$.

At step S102, there is an optional interact step to obtain for model 0, a mode state vector **x,** a covariance **P** and a mixing probability μ at time k-1. For *i* and *j*, the mixing probability $\mu_{j|i,k|k}$ at time *k* (the weights with which the estimates from the previous cycle k-1 are given to each filter at the beginning of the current cycle k) is defined as

$$\mu_{j|i,k-1|k-1} = \frac{p_{ji}\mu_{i,k-1}}{\overline{c}_j} \qquad (66)$$

where $\mu_{i,k}$ is the mode probability, i.e. probability of each model i being the true model, at time *k,* and $\overline{c}_j$ is a normalisation factor

$$\overline{c}_j = \sum_i p_{ji}\mu_{i,k-1} \qquad (67)$$

The model interaction for mode *j* proceeds as

$$\hat{\mathbf{x}}_{0j,k-1|k-1} = \sum_i \hat{\mathbf{x}}_{j,k-1|k-1}\mu_{j|i,k-1|k-1} \qquad (68)$$

$$\mathbf{P}_{0j,k-1|k-1} = \sum_i \left[ \mathbf{P}_{i,k-1|k-1} + \left( \hat{\mathbf{x}}_{i,k-1|k-1} - \hat{\mathbf{x}}_{0j,k-1|k-1} \right)\left( \hat{\mathbf{x}}_{i,k-1|k-1} - \hat{\mathbf{x}}_{0j,k-1|k-1} \right)^T \right]\mu_{j|i,k-1|k-1} \qquad (69)$$

At step S104, there is a predict step to obtain for each model j, an estimate for the mode state vector **x,** covariance **P** and mixing probability μ at time k based on the observations, up to and including time k-1. In other words, the predict step uses the state estimate from the previous timestep to produce an estimate of the state at the current timestep.

The predict step for mode *j* consists of

$$\hat{\mathbf{x}}_{j,k|k-1} = \mathbf{F}_k^0 + \mathbf{F}_k\hat{\mathbf{x}}_{0j,k-1|k-1} \qquad (70)$$

$$\mathbf{P}_{j,k|k-1} = \mathbf{F}_k\mathbf{P}_{0j,k-1|k-1}\mathbf{F}_k^T + \mathbf{Q}_{j,k} \qquad (71)$$

At step S106, there is an update step which uses measurement information at the current timestep k to refine for each model j, the estimates for the mode state vector **x,** covariance **P** and mixing probability μ for the current timestep obtained from step S104. The resulting estimates should be more accurate estimates for the current timestep.

In case of a glucose measurement $\tilde{g}_{1G,k}$, for each mode *j* the update consists of evaluating the measurement residual $\tilde{y}_{j,k}$

$$\tilde{y}_{j,k} = \mathbf{z}_k - \mathbf{H}\hat{\mathbf{x}}_{j,k|k-1} = \left[ \tilde{g}_{IG,k} - \frac{q_{j,3f,k|k-1} + F_{j,k|k-1}f_{12,10}}{V_G} \right] \qquad (72)$$

then evaluating the residual covariance $\mathbf{S}_{j,k}$

$$\mathbf{S}_{j,k} = \mathbf{H}\mathbf{P}_{j,k|k-1}\mathbf{H}^T + \mathbf{R}_k = \left[ \frac{p_{j,k|k-1,55} + f_{12,10}p_{j,k|k-1,45} + f_{12,10}\left(p_{j,k|k-1,54} + f_{12,10}p_{j,k|k-1,44}\right)}{V_G^2} + \sigma_{Z,k}^2 \right] \qquad (73)$$

The optimal Kalman gain is obtained as

$$\mathbf{K}_{j,k} = \mathbf{P}_{j,k|k-1}\mathbf{H}^T\mathbf{S}_{j,k}^{-1} = \mathbf{P}_{j,k|k-1}\frac{V_G}{d_j}\begin{bmatrix} 0 \\ 0 \\ 0 \\ f_{12,10} \\ 1 \end{bmatrix} = \frac{V_G}{d_j}\begin{bmatrix} p_{j,k|k-1,15} + f_{12,10}p_{j,k|k-1,14} \\ p_{j,k|k-1,25} + f_{12,10}p_{j,k|k-1,24} \\ p_{j,k|k-1,35} + f_{12,10}p_{j,k|k-1,34} \\ p_{j,k|k-1,45} + f_{12,10}p_{j,k|k-1,44} \\ p_{j,k|k-1,55} + f_{12,10}p_{j,k|k-1,54} \end{bmatrix} \qquad (74)$$

where

$$d_j = p_{j,k|k-1,55} + f_{12,10}p_{j,k|k-1,45} + f_{12,10}\left(p_{j,k|k-1,54} + f_{12,10}p_{j,k|k-1,44}\right) + \sigma_{Z,k}^2 V_G^2 \qquad (75)$$

The updated state estimate is obtained as

$$\hat{\mathbf{x}}_{j,k|k} = \hat{\mathbf{x}}_{j,k|k-1} + \mathbf{K}_{j,k}\tilde{y}_{j,k} = \hat{\mathbf{x}}_{j,k|k-1} + \frac{\tilde{g}_{IG,k}V_G - q_{j,3f,k|k-1} - F_{j,k|k-1}f_{12,10}}{d_j}\begin{bmatrix} p_{j,k|k-1,15} + f_{12,10}p_{j,k|k-1,14} \\ p_{j,k|k-1,25} + f_{12,10}p_{j,k|k-1,24} \\ p_{j,k|k-1,35} + f_{12,10}p_{j,k|k-1,34} \\ p_{j,k|k-1,45} + f_{12,10}p_{j,k|k-1,44} \\ p_{j,k|k-1,55} + f_{12,10}p_{j,k|k-1,54} \end{bmatrix}$$

$$(76)$$

The updated estimate covariance for the optimal Kalman gain is obtained as

$$\mathbf{P}_{j,k|k} = \left( I - \mathbf{K}_{j,k}\mathbf{H} \right)\mathbf{P}_{j,k|k-1} =$$

$$\begin{bmatrix} 1 & 0 & 0 & -f_{12,10}\dfrac{p_{j,k|k-1,15} + k_{12,10}p_{j,k|k-1,14}}{d_j} & -\dfrac{p_{j,k|k-1,15} + f_{12,10}p_{j,k|k-1,14}}{d_j} \\ 0 & 1 & 0 & -f_{12,10}\dfrac{p_{j,k|k-1,25} + k_{12,10}p_{j,k|k-1,24}}{d_j} & -\dfrac{p_{j,k|k-1,25} + f_{12,10}p_{j,k|k-1,24}}{d_j} \\ 0 & 0 & 1 & -f_{12,10}\dfrac{p_{j,k|k-1,35} + k_{12,10}p_{j,k|k-1,34}}{d_j} & -\dfrac{p_{j,k|k-1,35} + f_{12,10}p_{j,k|k-1,34}}{d_j} \\ 0 & 0 & 0 & 1-f_{12,10}\dfrac{p_{j,k|k-1,45} + k_{12,10}p_{j,k|k-1,44}}{d_j} & -\dfrac{p_{j,k|k-1,45} + f_{12,10}p_{j,k|k-1,44}}{d_j} \\ 0 & 0 & 0 & -f_{12,10}\dfrac{p_{j,k|k-1,55} + k_{12,10}p_{j,k|k-1,54}}{d_j} & 1-\dfrac{p_{j,k|k-1,55} + f_{12,10}p_{j,k|k-1,54}}{d_j} \end{bmatrix}\mathbf{P}_{j,k|k-1}$$

$$(77)$$

Alternatively, if $\mathbf{K}_{j,k}$ is not the optimal Kalman gain, the updated estimate covariance is obtained as

$$\mathbf{P}_{j,k|k} = \left( I - \mathbf{K}_{j,k}\mathbf{H} \right)\mathbf{P}_{j,k|k-1}\left( I - \mathbf{K}_{j,k}\mathbf{H} \right)^{T} + \mathbf{K}_{j,k}\mathbf{R}_k\mathbf{K}_{j,k}^{T} \qquad (78)$$

The likelihood function $\Lambda_{j,k}$ of mode $j$ is obtained as

$$\Lambda_{j,k} = \frac{V_G}{\sqrt{2\pi d_j}}\exp\left( -\frac{\tilde{y}_{j,k}^2 V_G^2}{2d_j} \right) \qquad (79)$$

and the mode probability as

$$\mu_{j,k} = \frac{1}{c}\Lambda_{j,k}\overline{c}_j \qquad (80)$$

Finally at step S108, there is a combine step whereby the estimated states and covariances for each model are combined to prove overall state and covariance estimates. The overall estimated state vector is obtained from a summation of all estimated state vectors multiplied by their weighting or mixing probability. Similarly, the estimated covariance is derived from a summation of all estimated covariances taking into account the relevant mixing probabilities.

$$\hat{\mathbf{x}}_{k|k} = \sum_{j}\hat{\mathbf{x}}_{j,k|k}\mu_{j,k} \qquad (81)$$

$$\mathbf{P}_{k-1|k-1} = \sum_{j}\left[ \mathbf{P}_{j,k|k} + \left( \hat{\mathbf{x}}_{j,k|k} - \hat{\mathbf{x}}_{k|k} \right)\left( \hat{\mathbf{x}}_{j,k|k} - \hat{\mathbf{x}}_{k|k} \right)^{T} \right]\mu_{j,k} \qquad (82)$$

Referring to Figure 1b, the control law used by the dose calculator to determine the correct dose is defined as

$$J = \sum_{j=N_1}^{N_2} \left[ \hat{y}_{t+j|t} - w_{t+j} \right]^2 + \lambda \sum_{j=1}^{N_2-d} \left[ \Delta u_{t+j-1} \right]^2 \qquad (83)$$

where $\hat{y}_{t+j|t}$ is plasma glucose concentration obtained using the combined step defined by Eq. (81) and interstitial glucose measurements taken up to time t, and

$$\Delta u_j = u_j - u_{j-1} \qquad (84)$$

The extended input vector $\mathbf{u}^+$ is defined as

$$\mathbf{u}^+ = \begin{pmatrix} v_t & \mathbf{u} \end{pmatrix}^T \qquad (85)$$

where $v_i$ denotes insulin bolus given at time i and
$\mathbf{u}$ is insulin infusion with the first element of $\mathbf{u}$, $u_1$, defining the insulin infusion rate to be delivered.

Thus the control law in matrix notation is

$$J = J_1 + J_2 = \left\| \mathbf{A}(\mathbf{u}^+ - \mathbf{u}_\mathbf{r}^+) + \mathbf{y}_\mathbf{r} - \mathbf{w} \right\| + \left\| \Lambda(\mathbf{u} - \mathbf{u}^{-1}) \right\| \quad (86)$$

where $\mathbf{w}$ is the set point (i.e. desired glucose level), $\mathbf{u_r}$ is the operating point, $\mathbf{y_r}$ is the output associated with the operating point, and A represents the model given by Eqs. (1) - (11) linearised around the operating point

$$a_{ij} = \frac{1}{V_G} \frac{dq_{1,j}}{du_i^+} \qquad (87)$$

with the matrix A defined as

$$\Lambda = \begin{pmatrix} \lambda_{t+1} & 0 & \cdots & \cdots & 0 \\ 0 & \lambda_{t+2} & & & \vdots \\ \vdots & & \ddots & & \vdots \\ \vdots & & & \lambda_{t+N-1} & 0 \\ 0 & \cdots & \cdots & 0 & \lambda_{t+N} \end{pmatrix} \qquad (88)$$

The two components of the control law $J_1$ and $J_2$ and their partial derivatives with respect to $\mathbf{u}^+$ can be written as

$$\begin{aligned} J_1 = \mathbf{u}^{+T}\mathbf{A}^T\mathbf{A}\mathbf{u}^+ &+ 2\left[ \left(\mathbf{y}_\mathbf{r} - \mathbf{w}\right)^T - \mathbf{u}_\mathbf{r}^{+T}\mathbf{A}^T \right]\mathbf{A}\mathbf{u} \\ &+ \left[ \mathbf{u}_\mathbf{r}^{+T}\mathbf{A}^T - 2\left(\mathbf{y}_\mathbf{r} - \mathbf{w}\right)^T \right]\mathbf{A}\mathbf{u}_\mathbf{r}^+ - 2\mathbf{w}^T\mathbf{y}_\mathbf{r} + \mathbf{y}_\mathbf{r}^T\mathbf{y}_\mathbf{r} + \mathbf{w}^T\mathbf{w} \end{aligned} \qquad (89)$$

$$J_2 = \left(\mathbf{u} - \mathbf{u}^{-1}\right)^T \Lambda^T \Lambda \left(\mathbf{u} - \mathbf{u}^{-1}\right) \qquad (90)$$

$$\frac{dJ_1}{d\mathbf{u}^+} = 2\mathbf{A}^T\mathbf{A}\mathbf{u}^+ + 2\mathbf{A}^T\left(\mathbf{y}_\mathbf{r} - \mathbf{w} - \mathbf{A}\mathbf{u}_\mathbf{r}^+\right) \qquad (91)$$

$$\frac{dJ_2}{d\mathbf{u}^+} = 2\left(\mathbf{B}^T\mathbf{B}\mathbf{u}^+ + \mathbf{b}\right) \qquad (92)$$

with

$$\mathbf{B} = \begin{pmatrix} 0 & 0 & \cdots & \cdots & \cdots & 0 \\ 0 & -\lambda_{t+1} & 0 & & & \vdots \\ 0 & \lambda_{t+2} & -\lambda_{t+2} & 0 & & \vdots \\ \vdots & 0 & & \ddots & & 0 \\ 0 & & 0 & \lambda_{t+N-1} & -\lambda_{t+N-1} & 0 \\ 0 & \cdots & \cdots & 0 & \lambda_{t+N} & -\lambda_{t+N} \end{pmatrix} \qquad (93)$$

$$\mathbf{B}^T\mathbf{B} = \begin{pmatrix} 0 & 0 & 0 & \cdots & \cdots & 0 \\ 0 & \lambda_{t+1}^2 + \lambda_{t+2}^2 & -\lambda_{t+2}^2 & 0 & & \vdots \\ 0 & -\lambda_{t+2}^2 & \lambda_{t+2}^2 + \lambda_{t+3}^2 & -\lambda_{t+3}^2 & & \vdots \\ \vdots & 0 & & \ddots & & 0 \\ 0 & & 0 & -\lambda_{t+N-2}^2 & \lambda_{t+N-2}^2 + \lambda_{t+N-1}^2 & -\lambda_{t+N}^2 \\ 0 & \cdots & \cdots & 0 & -\lambda_{t+N-1}^2 & \lambda_{t+N-1}^2 + \lambda_{t+N}^2 \end{pmatrix} \qquad (94)$$

and

$$\mathbf{b} = \begin{pmatrix} 0 & -\lambda_{t+1}^2 u_t & 0 & \cdots & 0 \end{pmatrix}^T \qquad (95)$$

The derivative of $J$ with respect to $\mathbf{u}^+$ is then obtained as

$$\frac{dJ}{d\mathbf{u}^+} = 2\left(\mathbf{C}\mathbf{u}^+ + \mathbf{c}\right) = 0 \qquad (96)$$

where

$$\mathbf{C} = \mathbf{A}^T\mathbf{A} + \mathbf{B}^T\mathbf{B} \qquad (97)$$

$$\mathbf{c} = \mathbf{A}^T\left(\mathbf{y}_r - \mathbf{w} - \mathbf{A}\mathbf{u}_r^+\right) + \mathbf{b} \qquad (98)$$

The solution is found as

$$\mathbf{u}^+ = -\mathbf{C}^{-1}\mathbf{c} \qquad (99)$$

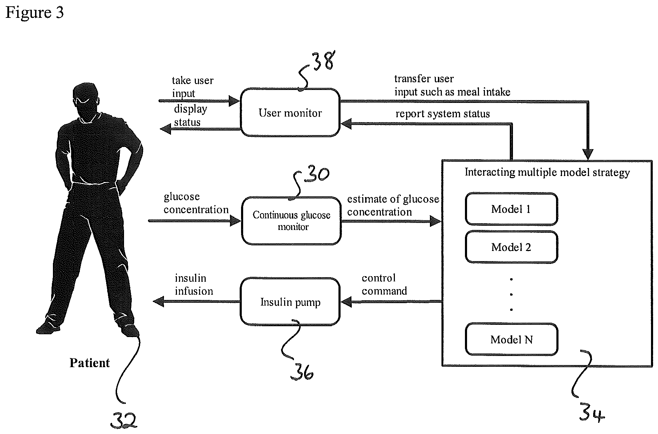

Figure 3 is a schematic showing apparatus implementing the method of controlling levels of glucose described above. The apparatus comprises at least one continuous glucose monitor 30 which measures the glucose concentration in a patient 32 at regular time intervals which may be every minute, every hour or another user defined interval. The glucose monitor may measure glucose levels intravenously, subcutaneously and/or intradermally. The measured glucose levels provide an initial estimate of the patient's inferred glucose level, e.g. plasma glucose concentration.

The measured glucose levels are input to a processor 34 which calculates a refined estimate of glucose level using the interacting multiple model strategy. The processor 34 uses the refined estimated to calculate a dose to be applied to the patient. The processor 34 is connected to an insulin pump 36 and delivers a control command to the insulin pump 36 in apply the calculated insulin infusion rate to the patient.

The apparatus also comprises an optional user monitor 38 which is connected to the processor 34. The user monitor 38 has an input interface to receive inputs from the user such as meal intake, exercise, etc. This information is input to the processor 34 to use when calculating the refined glucose estimate and dose. The user monitor 38 also receives system status information from the processor and has an output interface to display such information to the patient.

**Appendix**

This appendix describes the closed-form approximation of coefficients $f_{70}$, $f_{77}$, $f_{78}$, $f_{79}$, $f_{80}$, $f_{87}$, $f_{88}$, $f_{89}$, $f_{12,0}$, $f_{12,7}$, $f_{12,8}$, $f_{12,9}$, and $f_{12,12}$.

The first element of $\mathbf{u}$, $u_1$, defines the insulin infusion rate to be delivered. The amount of glucose appearing during the time interval $[t_{k-1}, t_k)$ can be approximated by a linear function $u_G(t)$ (mmol/kg/min) as

$$u_G(t) = u_{G,k-1} + \frac{u_{G,k} - u_{G,k-1}}{\Delta t_k} t + u_{S,k-1} \qquad (100)$$

where

$$u_{G,k} = \begin{cases} EGP_k - F_{01,k} + u_{A,k} & \text{if bioavailability is not estimated} \\ EGP_k - F_{01,k} & \text{if bioavailability is estimated} \end{cases} \qquad (101)$$

The fractional first order turnover rate $k_{01}$ from the accessible glucose compartment is obtained as a piecewise constant approximation during the time interval $\Delta t_k$

$$k_{01}(t) = k_{01,k} = S_{ID} \frac{x_D(t_{k-1}) + x_D(t_k)}{2} \qquad (102)$$

Denote $r_k$ the rate of change in $u_k$

$$r_k = \frac{u_{G,k} - u_{G,k-1}}{\Delta t_k} \qquad (103)$$

Define the auxiliary variables $v_1$ to $v_{18}$

$$v_1 = k_{01} + k_{12}$$

$$v_2 = (k_{01} - k_{12})^2 + k_{21}(2v_1 + k_{21})$$

$$v_3 = v_1 + k_{21}$$

$$v_4 = k_{01}k_{12}$$

$$v_5 = \sqrt{v_2}$$

$$v_6 = k_{12} - k_{31}$$

$$v_7 = k_{12} + k_{21}$$

$$v_8 = k_{12}(k_{12} + (k_{21} - k_{31})) + k_{21}k_{31} - k_{01}v_6$$

$$v_9 = 2v_2(v_4 - k_{31}(v_3 - k_{31}))$$

$$v_{10} = v_4(k_{21} - v_6) - k_{01}k_{21}(k_{31} - 2k_{21})$$

$$v_{11} = \frac{1}{v_5}(k_{01} - k_{12} + k_{21})$$

$$v_{12} = e^{-k_{31}\Delta t_k}$$

$$v_{13} = e^{-\frac{1}{2}\Delta t_k(v_3 + v_5)}$$

$$v_{14} = e^{\Delta t_k v_5}$$

$$v_{15} = v_{13}(v_{14} - 1)$$

$$v_{16} = v_{13}(v_{14} + 1) \tag{104}$$

$$v_{17} = v_2(v_6 + k_{21})$$

$$v_{18} = v_5(k_{01}(k_{21} - v_6) + v_7(v_6 + k_{21}))$$

Then

$$f_{77} = \frac{(v_{16} - v_{15}v_{11})}{2} \tag{105}$$

$$f_{87} = \frac{k_{21}v_{15}}{v_5} \tag{106}$$

$$f_{12,7} = \frac{k_{31}}{v_9}(v_{13}(-v_6v_2 + v_5v_8 - v_{14}(v_6v_2 + v_5v_8)) + 2v_2v_6v_{12}) \tag{107}$$

$$f_{78} = \frac{v_{15}k_{12}}{v_5} \tag{108}$$

$$f_{88} = \frac{(v_{16} + v_{15}v_{11})}{2} \tag{109}$$

$$f_{12,8} = \frac{k_{31}k_{12}}{v_9}(v_{13}(-v_2 + v_5(v_3 - 2k_{31}) - v_{14}(v_2 + v_5(v_3 - 2k_{31}))) + 2v_2v_{12}) \tag{110}$$

$$f_{79} = \frac{1}{k_{01}} \left[ 1 + \frac{v_{13}}{2v_5} (v_7 - k_{01} - v_5 + (k_{01} - v_5 - v_7)v_{14}) \right] (111)$$

$$f_{89} = \frac{k_{21}}{v_4} \left[ 1 + \frac{v_{13}}{2} \left( \frac{v_3}{v_5} - 1 - \left( \frac{v_3}{v_5} + 1 \right) v_{14} \right) \right] \quad (112)$$

$$f_{12,9} = \frac{1}{k_{01}} \left[ 1 + \frac{1}{v_9} (k_{31}v_{13}(v_{17} - v_{18} + (v_{17} + v_{18})v_{14}) - 2v_2 v_6 v_{12} k_{01}) \right] \quad (113)$$

The partial derivatives $g_1 = \partial q_{1,k} / \partial u_{G,k}$, $g_2 = \partial q_{2,k} / \partial u_{G,k}$, and $g_5 = \partial q_{3,k} / \partial u_{G,k}$ can be obtained as

$$g_1 = \frac{1}{\Delta t_k k_{01}} \left[ \Delta t_k - \frac{1}{v_4} \left( v_7 - \frac{v_{13}}{2v_5} (v_7(v_5 - v_7) + k_{01}(k_{12} - k_{21}) + (v_7(v_7 + v_5) + k_{01}(k_{21} - k_{12}))v_{14}) \right) \right]$$

$$(114)$$

$$g_2 = \frac{k_{21}}{\Delta t_k v_4} \left[ \Delta t_k - \frac{1}{v_4} \left( v_3 - \frac{v_{13}}{2v_5} ((v_5(2v_3 - v_7 - k_{01}) + 2v_4 - v_3^2) + (v_3(v_3 + v_5) - 2v_4)v_{14}) \right) \right]$$

$$(115)$$

$$g_5 = \frac{1}{(v_6(k_{01} - k_{31}) - k_{21}k_{31})\Delta t_k} \left[ \frac{v_9}{2v_2 k_{01}} \left( \Delta t_k - \frac{v_7 k_{31} + v_4}{v_4 k_{31}} \right) + \left( \frac{k_{31}v_{13}}{2v_4 v_5 k_{01}} (k_{01}k_{21}(k_{01} - v_5) + \right. \right.$$

$$+ (v_7 - v_5)v_7(v_6 + k_{21}) + v_{10} - v_{14}(k_{01}k_{21}(v_5 + k_{01}) + (v_5 + v_7)v_7(v_6 + k_{21}) + v_{10})) + \frac{v_{12}v_6}{k_{31}} \right]$$

$$(116)$$

These partial derivatives can be used to calculate other coefficients

$$f_{70} = f_{79}u_{G,k-1} + g_1 r_k \qquad (117)$$

$$f_{80} = f_{89}u_{G,k-1} + g_2 r_k \qquad (118)$$

$$f_{12,0} = f_{12,9}u_{G,k-1} + g_5 r_k \qquad (119)$$

$$f_{12,12} = v_{12} \qquad (120)$$

The coefficients $k_{7,10,k}$, $k_{8,10,k}$, and $k_{12,10,k}$ at time $t_k$ are calculated recursively using corresponding coefficients $k_{7,10,k-1}$, $k_{8,10,k-1}$, and $k_{12,10,k-1}$ obtained in the previous time instance $t_{k-1}$

$$f_{7,10} = f_{7,10,k-1}f_{77} + f_{8,10,k-1}f_{78} + f_{79}u_{A,k-1} + g_1 \left( u_{A,k} - u_{A,k-1} \right) \quad (121)$$

$$f_{8,10} = f_{8,10,k-1}f_{88} + f_{7,10,k-1}f_{87} + f_{89}u_{A,k-1} + g_2\left(u_{A,k} - u_{A,k-1}\right) \quad (122)$$

$$f_{12,10} = f_{12,10,k-1}f_{12,12} + f_{7,10,k-1}f_{12,7} + f_{8,10,k-1}f_{12,8} + f_{12,9}u_{A,k-1} + g_5\left(u_{A,k} - u_{A,k-1}\right) \quad (123)$$

**Claims**

1. Apparatus for monitoring glucose in human or animal in real time, the apparatus comprising:

   a sensor (30) providing a time series of measurements of glucose level, said measurements being indicative of an inferred level of said glucose in a part of said human or animal, wherein said inferred level comprises a level of said glucose in blood and said measurements comprise measurements of an interstitial glucose level and a processor which is adapted to perform the following steps:

   calculate a first estimate of said inferred glucose level from said measured glucose level using a first glucoregulatory model,
   calculate a second estimate of said inferred glucose level from said measured glucose level using a second glucoregulatory model with said second glucoregulatory model being a variation of said first glucoregulatory model and
   predict a combined estimate of the inferred glucose level based on a combination of the first and second estimates;
   wherein the first and second glucoregulatory models comprise a sub-model of glucose kinetics in the blood of said human or animal and a sub-model of interstitial glucose kinetics,
   wherein by using multiple models, an interacting multiple model strategy is being used, and
   wherein the processor is a state estimator and is adapted to define a state vector for each model, the state vector comprising a set of variables each having an associated uncertainty, the set of variables including a variable representing an unexplained change in glucose level with each model having a different standard deviation in the unexplained change in glucose level.

2. Apparatus according to claim 1, wherein the set of variables comprises variables representing glucose amounts in the accessible, non-accessible and/or interstitial compartments and the variable representing an unexplained change in glucose level.

3. Apparatus according to claim 1 or 2, wherein the processor is adapted to calculate a combined estimate by weighting each estimate according to an associated mixing probability representing a respective probability of each said model correctly predicting said glucose level.

4. Apparatus according to claim 3, wherein the processor is further adapted to update said mixing probability responsive to a difference between said predicted glucose level measurement of each respective said model and said glucose level measurement from said sensor

5. Apparatus according to any one of the preceding claims, further comprising a real-time alarm which is activated when the combined estimate of future glucose level is below a preset hypoglycaemia threshold or above a preset hyperglycaemia threshold.

6. Apparatus for real time control of glucose in a human or animal, the apparatus comprising:

   apparatus for monitoring glucose in human or animal in real time as described in any one of claims 1 to 5, and
   a dispenser (36) for delivering a specified amount of medication to a user in response to a command from the processor,
   wherein the processor is adapted to calculate the specified amount of medication as the amount of medication required to bring the combined estimate of the inferred glucose level measurement into line with a desired value.

7. Apparatus for controlling glucose in human or animal body, comprising
   apparatus for monitoring glucose in human or animal in real time as described in any one of claims 1 to 5, and
   a user interface for displaying a suggested insulin bolus to be applied

wherein the suggested insulin bolus is calculated by the processor as the amount of medication required to bring the combined estimate into line with a desired glucose value.

8. Apparatus according to claim 6 or claim 7, wherein the desired glucose varies with time to define a trajectory of values.

9. Apparatus according to any one of claims 6 to 8, wherein the processor applies an interacting multiple model strategy to the first and second glucoregulatory models to determine the amount of medication required.

10. A method of real time glucose monitoring in a living human or animal, the method comprising:

using a sensor to provide a time series of glucose level measurements from a sensor, said glucose level measurements being indicative of a inferred level of said glucose in a part of said human or animal, wherein said inferred level comprises a level of said glucose in blood and said measurements comprise measurements of an interstitial glucose level,
calculating a first estimate of said inferred glucose level from said measured glucose level using a first glucoregulatory model,
calculating a second estimate of said inferred glucose level from said measured glucose level using a second glucoregulatory model with said second glucoregulatory model being a variation of said first glucoregulatory model and
predicting a combined estimate of the inferred glucose level based on a combination of the first and second estimates
wherein the first and second glucoregulatory models comprise a sub-model of glucose kinetics in the blood of said human or animal and a sub-model of interstitial glucose kinetics,
wherein by using multiple models, an interacting multiple model strategy is being used, and
the method further comprising
defining a state vector for each model, the state vector comprising a set of variables each having an associated uncertainty, the set of variables including a variable representing an unexplained change in glucose level with each model having a different standard deviation in the unexplained change in glucose level.

11. A method according to claim 10, wherein each estimate is weighted according to a mixing probability representing a respective probability of each said model correctly predicting said glucose level when combining the multiple estimates.

12. A method according to claim 11, comprising updating said mixing probability responsive to a difference between said predicted glucose level measurement of each respective said model and said glucose level measurement from said sensor.

13. A method according to claim 12, wherein the glucoregulatory model further comprises a sub-model of insulin absorption, a sub-model of insulin action and a sub-model of gut absorption.

14. A method according to claim 13, comprising defining at time k-1 for each model a covariance which is a measure of the estimated accuracy of the state estimate and wherein the state vector is

$$\mathbf{x}_k^e = \left( i_{1,k}, i_{2,k}, r_{D,k}, r_{E,k}, a_{1,k}, a_{2,k}, q_{1,k}, q_{2,k}, q_{3,k}, u_{S,k} \right)^T$$

where $i_{1,k}$ and $i_{2,k}$ is the amount of insulin in the two subcutaneous insulin depots at time k, $\gamma_{D,k}$ and $\gamma_{E,k}$ are the remote insulin actions affecting glucose disposal and endogenous glucose production, $a_{1,k}$ and $a_{2,k}$ are the amount of glucose in the two absorption compartments, $q_{1,k}$, $q_{2,k}$, $q_{3,k}$ represent glucose amounts in the accessible, non-accessible, and interstitial compartments and $u_{s,k}$ is the unexplained glucose influx.

15. A carrier carrying computer program code to, when running, implementing the method of claims 10 to 14.

**Patentansprüche**

1. Gerät zur Überwachung von Glukose beim Menschen oder beim Tier in Echtzeit, wobei das Gerät Folgendes beinhaltet:

einen Sensor (30), welcher eine Zeitserie von Glukosespiegel-Messungen bereitstellt, wobei die Messungen für einen abgeleiteten Spiegel der Glukose in einem Körperteil des Menschen oder des Tiers stehen, wobei der abgeleitete Spiegel einen Spiegel der Glukose im Blut beinhaltet und die Messungen Messungen eines interstitiellen Glukosespiegels beinhalten, und

einen Prozessor, der geeignet ist, folgende Schritte auszuführen:

Berechnen eines ersten Schätzwertes des abgeleiteten Glukosespiegels anhand des gemessenen Glukosespiegels unter Verwendung eines ersten Glukoseregulationsmodells,
Berechnen eines zweiten Schätzwertes des abgeleiteten Glukosespiegels anhand des gemessenen Glukosespiegels unter Verwendung eines zweiten Glukoseregulationsmodells, wobei das zweite Glukoseregulationsmodell eine Variation des ersten Glukoseregulationsmodells ist und
Voraussagen eines kombinierten Schätzwertes des abgeleiteten Glukosespiegels auf der Grundlage einer Kombination des ersten und des zweiten Schätzwertes;
wobei das erste und das zweite Glukoseregulationsmodell ein Untermodell der Glukosekinetik im Blut des Menschen oder des Tiers und ein Untermodell der interstitiellen Glukosekinetik beinhalten, wobei bei Verwendung multipler Modelle eine interagierende multiple Modell-Strategie verwendet wird, und wobei der Prozessor ein Zustands-Schätzgerät ist, und geeignet ist, einen Zustandsvektor für jedes Modell zu definieren, wobei der Zustandsvektor einen Satz von Variablen beinhaltet, welche jeweils eine zugehörige Ungewissheit besitzen, wobei der Satz von Variablen eine Variable enthält, welche eine unerklärte Änderung des Glukosespiegels darstellt, wobei jedes Modell eine unterschiedliche Standardabweichung der unerklärten Änderung des Glukosespiegels besitzt.

2. Gerät nach Anspruch 1, bei welchem der Satz von Variablen Variablen beinhaltet, welche Glukosemengen in den zugänglichen, nichtzugänglichen und/oder interstitiellen Fächern darstellen, und die Variable, welche eine unerklärte Änderung des Glukosespiegels darstellt.

3. Gerät nach Anspruch 1 oder 2, bei welchem der Prozessor geeignet ist, einen kombinierten Schätzwert zu berechnen durch Gewichten eines jeden Schätzwertes nach einer dazugehörigen Misch-Wahrscheinlichkeit, welche eine jeweilige Wahrscheinlichkeit darstellt, mit welcher jedes der Modelle den Glukosespiegel korrekt vorhersagt.

4. Gerät nach Anspruch 3, bei welchem der Prozessor zudem geeignet ist, die Misch-Wahrscheinlichkeit zu aktualisieren, welche auf eine Differenz zwischen der vorhergesagten Glukosespiegelmessung eines jeden Modells und der Glukosespiegelmessung des Sensors anspricht.

5. Gerät nach einem der vorhergehenden Ansprüche, zudem beinhaltend einen Echtzeit-Alarm, der aktiviert wird, wenn der kombinierte Schätzwert von zukünftigen Glukosespiegeln unter einem voreingestellten Hypoglykämie-Grenzwert oder über einem voreingestellten Hyperglykämie-Grenzwert liegt.

6. Gerät zur Echtzeitprüfung von Glukose beim Menschen oder beim Tier, wobei das Gerät Folgendes beinhaltet:

Gerät zur Überwachung von Glukose beim Menschen oder beim Tier in Echtzeit wie in einem der Ansprüche 1 bis 5 beschrieben, und
einen Spender (36) zum Abgeben einer spezifizierten Menge an Medikation an einen Benutzer in Reaktion auf einen Steuerbefehl vom Prozessor,
wobei der Prozessor geeignet ist zum Berechnen der spezifizierten Menge an Medikation als der Menge an Medikation, welche benötigt wird, um den kombinierten Schätzwert der abgeleiteten Glukosespiegelmessung mit einem gewünschten Wert in Einklang zu bringen.

7. Gerät zur Prüfung von Glukose im menschlichen oder tierischen Körper, Folgendes beinhaltend:

Gerät zur Überwachung von Glukose beim Menschen oder beim Tier in Echtzeit wie in einem der Ansprüche 1 bis 5 beschrieben, und
eine Benutzerschnittstelle zum Anzeigen eines anzuwendenden vorgeschlagenen Insulin-Bolus
wobei der vorgeschlagene Insulin-Bolus vom Prozessor berechnet wird als die Menge an Medikation, welche benötigt wird, um den kombinierten Schätzwert mit einem gewünschten Glukosewert in Einklang zu bringen.

8. Gerät nach Anspruch 6 oder 7, bei welchem die gewünschte Glukose zeitlich schwankt, um eine Werte-Strecke zu definieren.

9. Gerät nach einem der Ansprüche 6 bis 8, bei welchem der Prozessor eine interagierende multiple Modell-Strategie auf das erste und das zweite Glukoseregulationsmodell anwendet, um die benötigte Menge an Medikation zu bestimmen.

10. Verfahren der Echtzeit-Glukoseüberwachung bei einem lebenden Menschen oder Tier, wobei das Verfahren Folgendes beinhaltet:

Verwenden eines Sensors zum Bereitstellen einer Zeitserie von Glukosespiegelmessungen von einem Sensor, wobei die Glukosespiegelmessungen für einen abgeleiteten Spiegel der Glukose in einem Körperteil des Menschen oder des Tiers stehen, wobei der abgeleitete Spiegel einen Spiegel der Glukose im Blut beinhaltet und die Messungen Messungen eines interstitiellen Glukosespiegels beinhalten,
Berechnen eines ersten Schätzwertes des abgeleiteten Glukosespiegels anhand des gemessenen Glukosespiegels unter Verwendung eines ersten Glukoseregulationsmodells,
Berechnen eines zweiten Schätzwertes des abgeleiteten Glukosespiegels anhand des gemessenen Glukosespiegels unter Verwendung eines zweiten Glukoseregulationsmodells, wobei das zweite Glukoseregulationsmodell eine Variation des ersten Glukoseregulationsmodells ist und
Voraussagen eines kombinierten Schätzwertes des abgeleiteten Glukosespiegels auf der Grundlage einer Kombination des ersten und des zweiten Schätzwertes;
wobei das erste und das zweite Glukoseregulationsmodell ein Untermodell der Glukosekinetik im Blut des Menschen oder des Tiers und ein Untermodell der interstitiellen Glukosekinetik beinhalten, wobei bei Verwendung multipler Modelle eine interagierende multiple Modell-Strategie verwendet wird, und
wobei das Verfahren zudem Folgendes beinhaltet:
Definieren eines Zustandsvektors für jedes Modell, wobei der Zustandsvektor einen Satz von Variablen beinhaltet, welche jeweils eine zugehörige Ungewissheit besitzen, wobei der Satz von Variablen eine Variable enthält, welche eine unerklärte Änderung des Glukosespiegels darstellt, wobei jedes Modell eine unterschiedliche Standardabweichung der unerklärten Änderung des Glukosespiegels besitzt.

11. Verfahren nach Anspruch 10, bei welchem jeder Schätzwert nach einer Misch-Wahrscheinlichkeit gewichtet wird, welche eine jeweilige Wahrscheinlichkeit darstellt, mit welcher jedes der Modelle den Glukosespiegel beim Kombinieren der multiplen Schätzwerte korrekt vorhersagt.

12. Verfahren nach Anspruch 11, beinhaltend das Aktualisieren der Misch-Wahrscheinlichkeit, welche auf eine Differenz zwischen der vorhergesagten Glukosespiegelmessung eines jeden Modells und der Glukosespiegelmessung des Sensors anspricht.

13. Verfahren nach Anspruch 12, bei welchem das Glukoseregulationsmodell zudem ein Untermodell der Insulinabsorption, ein Untermodell der Insulinwirkung und ein Untermodell der Darmabsorption beinhaltet.

14. Verfahren nach Anspruch 13, beinhaltend das Definieren einer Kovarianz zu einem Zeitpunkt k-1 für jedes Modell, welche eine Messung der geschätzten Genauigkeit der Zustandsschätzung ist, und wobei der Zustandsvektor wie folgt lautet:

$$\mathbf{x}_k^e = \left( i_{1,k}, i_{2,k}, r_{D,k}, r_{E,k}, a_{1,k}, a_{2,k}, q_{1,k}, q_{2,k}, q_{3,k}, u_{S,k} \right)^T$$

wobei $i_{1,k}$ und $i_{2,k}$ die Mengen an Insulin in den beiden subkutanen Insulindepots zum Zeitpunkt k sind, $\gamma_{D,k}$ und $\gamma_{E,k}$ die Insulin-Fernwirkungen sind, welche Glukosebeseitigung und endogene Glukoseproduktion beeinflussen, $a_{1,k}$ und $a_{2,k}$ die Glukosemengen in den beiden Absorptionsfächern sind, $q_{1,k}$, $q_{2,k}$, $q_{3,k}$ Glukosemengen in den zugänglichen, nichtzugänglichen und interstitiellen Fächern darstellen und $u_{s,k}$ der unerklärte Glukosezufluss ist.

15. Träger, welcher Computerprogrammcode trägt, um in Betrieb das Verfahren nach den Ansprüchen 10 bis 14 zu implementieren.

**Revendications**

1. Appareil pour surveiller le glucose chez un être humain ou un animal en temps réel, l'appareil comprenant :

un capteur (30) qui fournit une série temporelle de mesures du niveau de glucose, lesdites mesures étant indicatives d'un niveau inféré dudit glucose dans une partie dudit être humain ou animal, dans lequel ledit niveau inféré comprend un niveau dudit glucose dans le sang et lesdites mesures comprennent des mesures d'un niveau de glucose interstitiel ; et

un processeur qui est adapté de manière à ce qu'il réalise les étapes qui suivent :

le calcul d'une première estimation dudit niveau de glucose inféré à partir dudit niveau de glucose mesuré en utilisant un premier modèle glucorégulateur ;

le calcul d'une seconde estimation dudit niveau de glucose inféré à partir dudit niveau de glucose mesuré en utilisant un second modèle glucorégulateur, ledit second modèle glucorégulateur étant une variante dudit premier modèle glucorégulateur ; et

la prédiction d'une estimation combinée du niveau de glucose inféré sur la base d'une combinaison des première et seconde estimations ; dans lequel :

les premier et second modèles glucorégulateurs comprennent un sous-modèle de la cinétique du glucose dans le sang dudit être humain ou animal et un sous-modèle de la cinétique du glucose interstitiel ; dans lequel :

en utilisant de multiples modèles, une stratégie à multiples modèles en interaction est utilisée ; et dans lequel :

le processeur est un estimateur d'état et il est adapté de manière à ce qu'il définisse un vecteur d'état pour chaque modèle, le vecteur d'état comprenant un jeu de variables dont chacune présente une incertitude associée, le jeu de variables incluant une variable qui représente une variation inexpliquée du niveau de glucose, chaque modèle présentant un écart type différent pour la variation inexpliquée du niveau de glucose.

2. Appareil selon la revendication 1, dans lequel le jeu de variables comprend des variables qui représentent des quantités de glucose dans les compartiments accessible, non accessible et/ou interstitiel et la variable qui représente une variation inexpliquée du niveau de glucose.

3. Appareil selon la revendication 1 ou 2, dans lequel le processeur est adapté de manière à ce qu'il calcule une estimation combinée en pondérant chaque estimation conformément à une probabilité de mélange associée qui représente une probabilité respective de chaque dit modèle prédisant correctement ledit niveau de glucose.

4. Appareil selon la revendication 3, dans lequel le processeur est en outre adapté de manière à ce qu'il mette à jour ladite probabilité de mélange en réponse à une différence entre ladite mesure de niveau de glucose prédit de chaque dit modèle respectif et ladite mesure de niveau de glucose en provenance dudit capteur.

5. Appareil selon l'une quelconque des revendications qui précèdent, comprenant en outre une alarme temps réel qui est activée lorsque l'estimation combinée du niveau de glucose futur est en-deçà d'un seuil d'hypoglycémie préétabli ou au-delà d'un seuil d'hypoglycémie préétabli.

6. Appareil pour le contrôle en temps réel du glucose chez un être humain ou un animal, l'appareil comprenant :

l'appareil pour surveiller le glucose chez un être humain ou un animal en temps réel tel que décrit selon l'une quelconque des revendications 1 à 5 ; et

un dispositif de délivrance (36) pour délivrer une quantité spécifiée de médication à un utilisateur en réponse à une commande en provenance du processeur ; dans lequel :

le processeur est adapté de manière à ce qu'il calcule la quantité spécifiée de médication en tant que quantité de médication requise pour amener l'estimation combinée de la mesure de niveau de glucose inféré en ligne avec une valeur souhaitée.

7. Appareil pour contrôler le glucose au niveau de l'organisme d'un être humain ou d'un animal, comprenant :

l'appareil pour surveiller le glucose chez un être humain ou un animal en temps réel tel que décrit selon l'une quelconque des revendications 1 à 5 ; et

une interface utilisateur pour afficher un bolus d'insuline suggéré qui doit être appliqué ; dans lequel :

le bolus d'insuline suggéré est calculé par le processeur en tant que quantité de médication requise pour amener l'estimation combinée en ligne avec une valeur de glucose souhaitée.

**8.** Appareil selon la revendication 6 ou la revendication 7, dans lequel le glucose souhaité varie en fonction du temps de sorte qu'il définit une trajectoire de valeurs.

**9.** Appareil selon l'une quelconque des revendications 6 à 8, dans lequel le processeur applique une stratégie à multiples modèles en interaction aux premier et second modèles glucorégulateurs de manière à déterminer la quantité de médication requise.

**10.** Procédé de surveillance du glucose en temps réel chez un être humain ou un animal vivant, le procédé comprenant :

l'utilisation d'un capteur pour obtenir une série temporelle de mesures de niveau de glucose à partir d'un capteur, lesdites mesures de niveau de glucose étant indicatives d'un niveau inféré dudit glucose dans une partie dudit être humain ou animal, dans lequel ledit niveau inféré comprend un niveau dudit glucose dans le sang et lesdites mesures comprennent des mesures d'un niveau de glucose interstitiel ;

le calcul d'une première estimation dudit niveau de glucose inféré à partir dudit niveau de glucose mesuré en utilisant un premier modèle glucorégulateur ;

le calcul d'une seconde estimation dudit niveau de glucose inféré à partir dudit niveau de glucose mesuré en utilisant un second modèle glucorégulateur, ledit second modèle glucorégulateur étant une variante dudit premier modèle glucorégulateur ; et

la prédiction d'une estimation combinée du niveau de glucose inféré sur la base d'une combinaison des première et seconde estimations ; dans lequel :

les premier et second modèles glucorégulateurs comprennent un sous-modèle de la cinétique du glucose dans le sang dudit être humain ou animal et un sous-modèle de la cinétique du glucose interstitiel ; dans lequel :

en utilisant de multiples modèles, une stratégie à multiples modèles en interaction est utilisée ; et le procédé comprenant en outre :

la définition d'un vecteur d'état pour chaque modèle, le vecteur d'état comprenant un jeu de variables dont chacune présente une incertitude associée, le jeu de variables incluant une variable qui représente une variation inexpliquée du niveau de glucose, chaque modèle présentant un écart type différent pour la variation inexpliquée du niveau de glucose.

**11.** Procédé selon la revendication 10, dans lequel chaque estimation est pondérée conformément à une probabilité de mélange qui représente une probabilité respective de chaque dit modèle prédisant correctement ledit niveau de glucose lors de la combinaison des multiples estimations.

**12.** Procédé selon la revendication 11, comprenant la mise à jour de ladite probabilité de mélange en réponse à une différence entre ladite mesure de niveau de glucose prédit de chaque dit modèle respectif et ladite mesure de niveau de glucose en provenance dudit capteur.

**13.** Procédé selon la revendication 12, dans lequel le modèle glucorégulateur comprend en outre un sous-modèle de l'absorption de l'insuline, un sous-modèle de l'action de l'insuline et un sous-modèle de l'absorption intestinale.

**14.** Procédé selon la revendication 13, comprenant la définition, à un temps k-1 pour chaque modèle, d'une covariance qui est une mesure de la précision estimée de l'estimation d'état et dans lequel le vecteur d'état est :

$$\mathbf{x}_k^e = \left( i_{1,k}, i_{2,k}, r_{D,k}, r_{E,k}, a_{1,k}, a_{2,k}, q_{1,k}, q_{2,k}, q_{3,k}, u_{S,k} \right)^T$$

où $i_{1,k}$ et $i_{2,k}$ sont les quantités d'insuline dans les deux sites de dépôt d'insuline sous-cutanés à un temps k, $\gamma_{D,k}$ et $\gamma_{E,k}$ sont les actions de l'insuline à distance qui affectent l'élimination du glucose et la production endogène du glucose, $a_{1,k}$ et $a_{2,k}$ sont les quantités de glucose dans les deux compartiments d'absorption, $q_{1,k}$, $q_{2,k}$ et $q_{3,k}$ représentent les quantités de glucose dans les compartiments accessible, non accessible et interstitiel et $u_{s,k}$ est l'influx de glucose inexpliqué.

**15.** Support porteur d'un code de programme informatique pour, lorsqu'il est exécuté, mettre en oeuvre le procédé des revendications 10 à 14.

Figure 1a.

Figure 1b.

Figure 2.

Mode state vector, covariance, and probability at *k-1|k-1* — S100

$$\hat{\mathbf{x}}_{i,k-1|k-1}$$

$$\mathbf{P}_{i,k-1|k-1}$$

$$\mu_{i,k-1}$$

$p_{ji}$

**Interact step** to obtain mode state vector, covariance, and probability at *0,k-1|k-1* — S102

$$\bar{c}_j = \sum_i p_{ji}\mu_{i,k-1}$$

$$\mu_{j|i,k-1|k-1} = \frac{p_{ji}\mu_{i,k-1}}{\bar{c}_j}$$

$$\hat{\mathbf{x}}_{0j,k-1|k-1} = \sum_i \hat{\mathbf{x}}_{j,k-1|k-1}\mu_{j|i,k-1|k-1}$$

$$\mathbf{P}_{0j,k-1|k-1} = \sum_i \left[ \mathbf{P}_{i,k-1|k-1} + \left( \hat{\mathbf{x}}_{i,k-1|k-1} - \hat{\mathbf{x}}_{0j,k-1|k-1} \right) \right.$$

$$\left. \left( \hat{\mathbf{x}}_{i,k-1|k-1} - \hat{\mathbf{x}}_{0j,k-1|k-1} \right)^T \right]\mu_{j|i,k-1|k-1}$$

$\mathbf{F}_k$

**Update step** to obtain mode state vector, covariance, and probability at *k|k*

$$\tilde{y}_{j,k} = \mathbf{z}_k - \mathbf{H}\hat{\mathbf{x}}_{j,k|k-1}$$

$$\mathbf{S}_{j,k} = \mathbf{H}\mathbf{P}_{j,k|k-1}\mathbf{H}^T + \mathbf{R}_k$$

$$\mathbf{K}_{j,k} = \mathbf{P}_{j,k|k-1}\mathbf{H}^T\mathbf{S}_{j,k}^{-1}$$

$$\hat{\mathbf{x}}_{j,k|k} = \hat{\mathbf{x}}_{j,k|k-1} + \mathbf{K}_{j,k}\tilde{y}_{j,k}$$

$$\mathbf{P}_{j,k|k} = \left( \mathbf{I} - \mathbf{K}_{j,k}\mathbf{H} \right)\mathbf{P}_{j,k|k-1}$$

$$\Lambda_{j,k} = \frac{V_G}{\sqrt{2\pi d_j}}\exp\left( -\frac{\tilde{y}_{j,k}^2 V_G^2}{2d_j} \right)$$

$$\mu_{j,k} = \frac{1}{c}\Lambda_{j,k}\bar{c}_j$$

— S106

$\mathbf{z}_k$

**Predict step** to obtain mode state vector, covariance, and probability at *k|k-1*

$$\hat{\mathbf{x}}_{j,k|k-1} = \mathbf{F}_k^0 + \mathbf{F}_k\hat{\mathbf{x}}_{0j,k-1|k-1}$$

$$\mathbf{P}_{j,k|k-1} = \mathbf{F}_k\mathbf{P}_{0j,k-1|k-1}\mathbf{F}_k^T + \mathbf{Q}_{j,k}$$

— S104

**Combine step** to obtain combined state and covariance estimate at *k|k*

$$\hat{\mathbf{x}}_{k|k} = \sum_j \hat{\mathbf{x}}_{j,k|k}\mu_{j,k}$$

$$\mathbf{P}_{k|k} = \sum_j \left[ \mathbf{P}_{j,k|k} + \left( \hat{\mathbf{x}}_{j,k|k} - \hat{\mathbf{x}}_{k|k} \right)\left( \hat{\mathbf{x}}_{j,k|k} - \hat{\mathbf{x}}_{k|k} \right)^T \right]\mu_{j,k}$$

— S108

Figure 3

EP 2 223 251 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5086229 A
- US 5497772 A
- US 6575905 B
- US 6572545 B
- WO 0224065 A
- WO 9728787 A
- US 4055175 A
- US 4464170 A
- US 20030208113 A
- WO 2005041103 A
- US 5325098 A
- US 7079991 B
- US 6876925 B
- EP 1278564 A
- EP 1725278 A

### Non-patent literature cited in the description

- **D. C. KLONOFF.** Continuous Glucose Monitoring: Roadmap for 21st century diabetes therapy. *Diabetes Care,* 2005, vol. 28 (5), 1231-1239
- **K. REBRIN ; G. M. STEIL ; W. P. VAN ANTWERP ; J. J. MASTROTOTARO.** Subcutaneous glucose predicts plasma glucose independent of insulin: implications for continuous monitoring. *American Journal of Physiology-Endocrinology and Metabolism,* 1999, vol. 277 (3), E561-E571
- **E. J. KNOBBE ; B. BUCKINGHAM.** The extended Kalman filter for continuous glucose monitoring. *Diabetes Technol.Ther.,* 2005, vol. 7 (1), 15-27
- **R. HOVORKA.** Continuous glucose monitoring and closed-loop systems. *Diabet.Med.,* 2006, vol. 23 (1), 1-12
- **R. S. PARKER ; F. J. DOYLE, III ; N. A. PEPPAS.** The intravenous route to blood glucose control. *IEEE Eng.Med.Biol.Mag.,* 2001, vol. 20 (1), 65-73
- **G. M. STEIL ; K. REBRIN ; C. DARWIN ; F. HARIRI ; M. F. SAAD.** Feasibility of automating insulin delivery for the treatment of type 1 diabetes. *Diabetes,* 2006, vol. 55 (12), 3344-3350
- **R. HOVORKA ; V. CANONICO ; L. J. CHASSIN ; U. HAUETER ; M. MASSI-BENEDETTI ; FEDERICI M. ORSINI ; T. R. PIEBER ; H. C. SCHALLER ; L. SCHAUPP ; T. VERING.** Nonlinear model predictive control of glucose concentration in subjects with type 1 diabetes. *Physiol Meas.,* 2004, vol. 25 (4), 905-920
- **R. S. PARKER ; F. J. DOYLE, III ; N. A. PEPPAS.** A model-based algorithm for blood glucose control in type I diabetic patients. *IEEE Trans.Biomed.Eng,* 1999, vol. 46 (2), 148-157
- **WILINSKA et al.** Interstitial glucose kinetics in subjects with Type 1 diabetes under physiologic conditions. *Metabolism,* 2004, vol. 53 (11), 1484-1492
- **E. MAZOR ; A. AVERBUCH ; Y. BAR-SHALOM ; J. DAYAN.** Interacting multiple model methods in target tracking: A survey. *IEEE Transactions on Aerospace and Electronic Systems,* 1998, vol. 34 (1), 103-123
- **D. S. BAYARD ; R. W. JELLIFFE.** A Bayesian approach to tracking patients having changing pharmacokinetic parameters. *J.Pharmacokinet.Pharmacodyn.,* 2004, vol. 31 (1), 75-107
- **P. ABOLMAESUMI ; M. R. SIROUSPOUR.** An interacting multiple model probabilistic data association filter for cavity boundary extraction from ultrasound images. *IEEE Trans.Med.Imaging,* 2004, vol. 23 (6), 772-784
- **D. A. LINKENS ; M. MAHFOUF.** Generalized Predictive Control with Feedforward (Gpcf) for Multivariable Anesthesia. *International Journal Of Control,* 1992, vol. 56 (5), 1039-1057
- **M. MAHFOUF ; D. A. LINKENS.** Non-linear generalized predictive control (NLGPC) applied to muscle relaxant anaesthesia. *International Journal Of Control,* 1998, vol. 71 (2), 239-257
- **M. M. STRUYS ; E. P. MORTIER ; SMET T. DE.** Closed loops in anaesthesia. *Best.Pract.Res.Clin.Anaesthesiol.,* 2006, vol. 20 (1), 211-220
- **V SARTORI ; P SCHUMACHER ; T BOUILLON ; M LUGINBUEHL ; M MORARI.** On-line estimation of propofol pharmacodynamic parameters. *Annual International Conference of the IEEE Engineering in Medicine and Biology Society,* 2005, vol. 1, 74-7